**Europäisches Patentamt**

**European Patent Office**

**Office européen des brevets**

(19)

(11) Veröffentlichungsnummer : **0 071 974
B1**

# EUROPÄISCHE PATENTSCHRIFT

(12)

(45) Veröffentlichungstag der Patentschrift :
**12.11.86**

(21) Anmeldenummer : **82107046.3**

(22) Anmeldetag : **04.08.82**

(51) Int. Cl.⁴ : **A 61 L 15/03, A 61 K 9/06**

(54) Antimikrobielle Zubereitungen zur Behandlung nässender offener Wunden.

(30) Priorität : **06.08.81 DE 3131112**

(43) Veröffentlichungstag der Anmeldung :
**16.02.83 Patentblatt 83/07**

(45) Bekanntmachung des Hinweises auf die Patenterteilung : **12.11.86 Patentblatt 86/46**

(84) Benannte Vertragsstaaten :
**AT BE CH DE FR GB IT LI LU NL SE**

(56) Entgegenhaltungen :
**GB-A- 1 516 653
H.P. FIEDLER et al.: "Lexikon der Hilfsstoffe für
Pharmazie, Kosmetik und angrenzende Gebiete",
Band 9, Seite 449, Editio Cantor KG., Aulendorf i.
Württ., DE.
Die Akte enthält technische Angaben, die nach dem
Eingang der Anmeldung eingereicht wurden und die
nicht in dieser Patentschrift enthalten sind.**

(73) Patentinhaber : **Intermedicat GmbH
Gerliswilstrasse 45
CH-6020 Emmenbrücke (CH)**

(72) Erfinder : **Schröder, Jürgen, Dr.
Auf dem Rottheil 7
D-3509 Spangenberg (DE)**
Erfinder : **Stabi, Harald
Kehrenbergstrasse 7
D-3501 Körle (DE)**

(74) Vertreter : **von Kreisler, Alek, Dipl.-Chem. et al
Deichmannhaus am Hauptbahnhof
D-5000 Köln 1 (DE)**

## Beschreibung

Der Einsatz von Polyäthylenglykolen als Salbengrundlage ist seit langem bekannt. Die nachgewiesene Unschädlichkeit von Polyäthylenglykolen hat dazu geführt, daß diese bereits seit langem in den verschiedenen Pharmakopöen verzeichnet sind. Polyäthylenglykole mit dem mittleren Molekulargewicht 300, 400, 1 500, 4 000 und 6 000 entsprechen z. B. den Monographien des DAB. Gemische von Polyäthylenglykolen der genannten mittleren Molekulargewichte werden als Salben sowohl für die Anwendung auf der Haut als auch zur Wundbehandlung eingesetzt. Gerade bei der Wundbehandlung ist der Einsatz von Polyäthylenglykolen als Salbengrundlage besonders vorteilhaft, da aufgrund der vollständigen Wasserlöslichkeit der Polyäthylenglykole keine Rückstände auf der Wunde verbleiben.

Einer der vielen antimikrobiellen Wirkstoffe, welche sich mit Polyäthylenglykolen vorteilhaft zu einer Salbe für die Wundbehandlung verarbeiten lassen, ist PVP-Jod. Hierbei handelt es sich um eine Anlagerung von Jod an Polyvinylpyrrolidon. Durch diese Anlagerung des Jods an PVP wird die lokale Reizwirkung drastisch herabgesetzt, während die antimikrobielle Wirkung vollständig erhalten bleibt.

Antimikrobielle Salben mit Polyäthylenglykolen der oben genannten Molekulargewichte und PVP-Jod haben sich daher seit langem in der Wundbehandlung, insbesondere bei großflächig Brandverletzten, besonders bewährt.

Überraschenderweise wurde nun gefunden, daß eine besonders gute Heilung erzielt werden kann, wenn die Salbengrundlage der antimikrobiellen Salbe zur Behandlung großflächiger Wunden Polyäthylenglykol enthält, dessen mittleres Molekulargewicht erheblich über den in den Pharmakopöen genannten Werten und erheblich über demjenigen der in der Praxis benutzten Polyäthylenglykole liegt.

Dementsprechend betrifft die Erfindung eine antimikrobielle Salbe, gekennzeichnet durch einen Gehalt an Polyethylenglykol mit einem mittleren Molekulargewicht von 8 000 und darüber, sowie einem Polyethylenglykol-Gehalt von 5 bis 50 Gew.-%.

Bevorzugt wird dabei ein mittleres Molekulargewicht von 20 000.

Die mittleren Molekulargewichte der erfindungsgemäss verwendeten Polyäthylenglykole sind erheblich höher als die in den gängigen Pharmakopöen beschriebenen Polyäthylenglykole mit einem mittleren Molekulargewicht von 6 000.

Die erfindungsgemässe Verwendung der Polyäthylenglykole mit einem mittlerem Molekulargewicht von 8 000 und darüber in der antimikrobiellen Salbe ergibt ein verbessertes Viskositätsverhalten, d. h. die Salbe zeigt bei der Verdünnung durch das Wundsekret eine geringere Fließneigung. Auch wenn das Polyäthylenglykol durch die offene Wunde zumindest teilweise resorbiert werden sollte, so ist bei einem mittleren Molekulargewicht des Polyäthylenglykols von 8 000 bis 40 000, z. B von 20 000, die vollständige Ausscheidung durch die Niere des Patienten gewährleistet.

Es liegt außerdem nahe und ist zu vermuten, daß bei noch höheren Molekulargewichten des Polyäthylenglykols, z. B. von 40 000 an ansteigend bis z. B. 100 000, keine Resorption durch den Kontakt der Salbe mit dem Wundgewebe erfolgt und damit auch keine Notwendigkeit besteht, das Polyäthylenglykol durch die Nieren ausscheiden zu müssen. Dies bedeutet aber, daß auch Polyäthylenglykole mit höherem Molekulargewicht als 40 000 — wenn dies gewünscht wird — erfindungsgemäss verwendbar sind. Bevorzugt wird jedoch ein Bereich des Molekulargewichts des Polyäthylenglykols von 8 000 bis 40 000 und insbesondere von ungefähr 20 000.

Der erfindungsgemässe Gehalt an Polyäthylenglykol beträgt von 5 bis 50 Gew.-%. Niedrigere Mengen zeigen kaum noch die gewünschte Wirkung, bei höheren Mengen sinkt der Gehalt an wirksamen antimikrobiellen Substanzen.

Wie aus dem folgenden Vergleichsversuch hervorgeht, ist die erfindungsgemässe Salbe der bekannten Salbe in ihrer Heilwirkung überlegen.

Vergleichsversuch

Bei 10 Wistar-Ratten mit einem Gewicht von ca 250 g wurden unter Äthernarkose Hautdefekte von 3 cm Durchmesser gesetzt. Danach wurde 1 ml einer Kultur von Pseudomonos aeroginosa, die $1 \times 10^9$ Keime/ml enthielt, auf die Wunde getröpft. Die Tiere wurden dann in zwei Gruppen eingeteilt. Bei Gruppe 1 wurde eine handelsübliche PVP-Jod-Salbe auf der Basis der in den Pharmakopöen genannten Polyäthylenglykole zwei Mal täglich aufgetragen. In Gruppe 2 wurde eine PVP-Jod-Salbe aufgetragen, die 30 % Polyäthylenglykol mit einem mittleren Molekulargewicht von 20 000 enthielt. Während die Wundheilung in Gruppe 1 nach 33 Tagen abgeschlossen war, war dies bei der Gruppe 2 schon nach 25 Tagen der Fall.

Die erfindungsgemässe antimikrobielle Salbe kann natürlich außer oder anstelle von PVP-Jod andere bekannte antimikrobielle Stoffe enthalten, wie z. B. Taurolin, Silber-Sulfadiazin u. a.

Die Herstellung der erfindungsgemässen Salbe erfolgt nach üblichen Verfahren, die dem Fachmann ohne weiteres bekannt sind.

## Patentanspruch

Antimikrobielle Salbe, gekennzeichnet durch einen Gehalt an Polyethylenglykol mit einem mittleren Molekulargewicht von 8 000 und darüber, sowie einem Polyethylenglykol-Gehalt von 5 bis 50 Gew.-%.

**Claim**

Antimicrobial ointment, characterized by a content of polyethylene glycol with an average molecular weight of 8 000 and more, and a polyethylene glycol content of 5 to 50 % by weight.

**Revendication**

Pommade antimicrobienne, caractérisée en ce qu'elle contient un polyéthylèneglycol ayant une masse moléculaire moyenne de 8 000 et plus, avec une teneur en polyéthylèneglycol de 5 à 50 % en poids.